# EUROPEAN PATENT APPLICATION

(11) **EP 0 847 987 A1**
(43) Date of publication of application: **17.06.1998**
(21) Application number: 97121746.8
(22) Date of filing: 10.12.1997
(51) Int. Cl.: C07C 231/02, C07C 233/02, C07C 233/45, C07C 229/08, C07C 227/18, C07C 227/22

(54) **Process for preparing amide derivatives, aminocarboxylic acid ester salts and their preparation**

(30) Priority: 10.12.1996 JP 329728/96; 10.12.1996 JP 329729/96
(71) Applicant: KAO CORPORATION, Chuo-ku Tokyo (JP)
(72) Inventor: Yoshino, Koji, Haga-gun, Tochigi (JP); Sugai, Yoshiya, Haga-gun, Tochigi (JP); Yamamuro, Akira, Haga-gun, Tochigi (JP)
(74) Representative: Hansen, Bernd, Dr. Dipl.-Chem.

(57) **Abstract**

The present invention provides a process for the manufacture of an amide derivative having the formula (4): 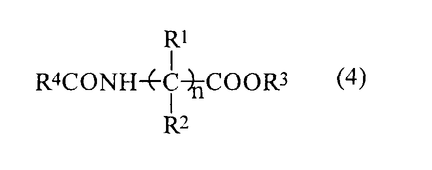 Since the intermediate product, an aminocarboxylic acid ester salt, is a liquid compound or is freely soluble in a solvent, the process does not pass through a solid compound, and thus the amide derivative can be produced industrially with ease.

## Description

### BACKGROUND OF THE INVENTION

### Field of the Invention:

The present invention relates to a novel process for preparing amide derivatives, and more particularly to a process for preparing N-acylaminocarboxylic acid esters through the use of an aminocarboxylic acid or a cyclic lactam as a starting material.

### Description of the Related Art:

Conventionally, amide derivatives, such as N-acylaminocarboxylic acid esters, have been used as starting materials in the manufacture of a variety of industrially useful compounds. In methods for preparing these amide derivatives, aminocarboxylic acid has been used as a starting material. Specifically, there have been employed the following methods: (1) a method in which aminocarboxylic acid is subjected to acylation in water or water-alcohol in the presence of sodium hydroxide or a similar substance (Schotten-Baumann method), followed by esterification; (2) a method in which aminocarboxylic acid is first esterified by use of an acid catalyst, and subsequently the acid catalyst is neutralized to collect a free aminocarboxylic ester, which is then subjected to acylation; and (3) a method in which aminocarboxylic acid is first esterified by use of an acid catalyst, and subsequently there is collected an aminocarboxylic ester salt in which the amino group has been neutralized with the acid, which is then subjected to acylation. Although method (1) is advantageous for the production of highly water-soluble aminocarboxylic acids such as amino acids, it has a drawback when used to prepare less water-soluble aminocarboxylic acids such as ω-aminocarboxylic acids having an Increased number of carbons, in that the resultant product precipitates in the reaction system, making the operation intricate and difficult. Method (2) cannot achieve high yields because the resultant free aminocarboxylic esters are highly prone to become polymerized. Method (3) is problematic in that the production steps are complicated because the resultant aminocarboxylic ester salt is a solid having a high melting point.

As described above, there have hitherto been known no industrially suitable processes that allow the inexpensive and simple manufacture of N-acylaminocarboxylic acid esters.

### SUMMARY OF THE INVENTION

In view of the foregoing, the present inventors have carried out careful studies and have found that when an aminocarboxylic acid or a cyclic lactam is esterified in the presence of a specified acid catalyst and the resultant esterified product is directly subjected to removal of excess alcohol, an aminocarboxylic acid ester salt is obtained which is in a liquid state between normal ambient temperature and about 100°C, and that when this product is subsequently acylated, a corresponding amide compound is produced at reduced cost and with ease without passing through a solid compound intermediate.

Accordingly, an object of the present invention is to provide a process for manufacturing amide derivatives, which process eliminates formation of a solid compound in an intermediate process when the amino group of the aminocarboxylic acid is acylated to form an amide bond.

The first embodiment of the present invention relates to a process for preparing an N-acylaminocarboxylic acid ester of the formula (4): 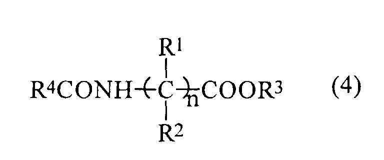 wherein R¹ and R², which may be identical to or different from each other, represent a hydrogen atom or a C₁-C₆ linear alkyl group, R³ represents a C₂-C₄₀ linear, branched, or cyclic alkyl group, R⁴CO- represents a group derived from a carboxylic acid halide or an acid anhydride, and n represents a number between 1 and 16 inclusive that includes:
reacting an aminocarboxylic acid of formula (1): 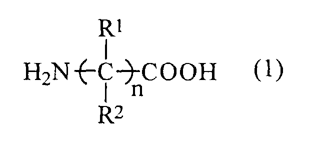
wherein R¹, R², and n have the same meanings as defined above,
or a cyclic lactam of formula (2): 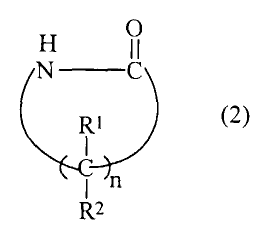
wherein R¹, R², and n have the same meanings as defined above,
with a monohydric alcohol R³OH having 2-40 carbon atoms in the presence of a divalent or trivalent inorganic acid;
removing unreacted alcohol thereby forming a resultant product; and
reacting the resultant product with a carboxylic acid halide or an acid anhydride of formula R⁴COX wherein X is a halogen atom or an acid anhydride residue (R⁴COO-) in the presence of a tertiary amine.

The second embodiment of the present invention relates to an aminocarboxylic acid ester salt having the formula (3): 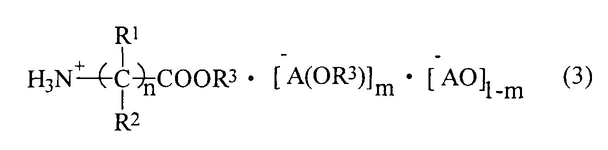 wherein R¹ and R², which may be identical to or different from each other, represent a hydrogen atom or a C₁-C₆ linear alkyl group, R³ represents a C₂-C₄₀ linear, branched, or cyclic alkyl group, ⁻A(OR³), wherein R³ has the same meaning as described above, represents an inorganic acid ester from which a single hydrogen ion has been released, -AO represents a divalent or trivalent acid residue from which a single hydrogen ion has been released, n represents a number between 1 and 16 inclusive, and m represents a number between 0 and 1 inclusive.

The third embodiment of the present inventin relates to a process for preparing an aminocarboxylic acid ester salt having the formula (3): 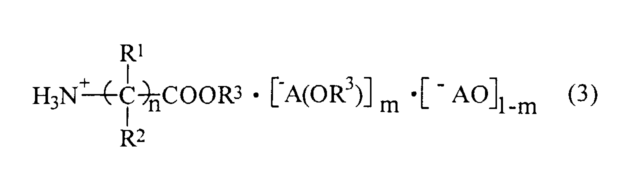 wherein R¹ and R², which may be identical to or different from each other, represent a hydrogen atom or a C₁-C₆ linear alkyl group, R³ represents a C₂-C₄₀ linear, branched, or cyclic alkyl group, ⁻A(OR³), wherein R³ has the same meaning as described above, represents an inorganic acid ester from which a single hydrogen ion has been released, ⁻AO represents a divalent or trivalent acid residue from which a single hydrogen ion has been released, n represents a number between 1 and 16 inclusive, and m represents a number between 0 and 1 inclusive, that includes;
reacting an aminocarboxylic acid of formula (1): 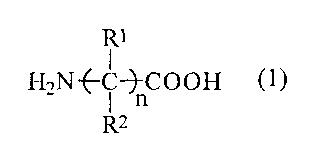
wherein R¹ and R², which may be identical to or different from each other, represent a hydrogen atom or a C₁-C₆ linear alkyl group, and n represents a number between 1 and 16 inclusive or a cyclic lactam of formula (2): 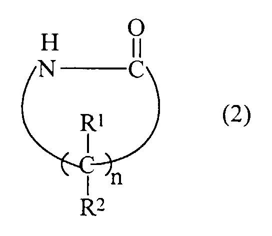
wherein R¹, R², and n have the same meanings as defined above, with a monohydric alcohol R³OH having 2-40 carbon atoms in the presence of a divalent or trivalent inorganic acid; and
removing unreacted alcohol.

The fourth embodiment of the present invention relates to a process for preparing an N-acylaminocarboxylic acid ester of the formula (4): 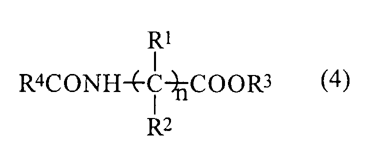 wherein R¹ and R², which may be identical to or different from each other, represent a hydrogen atom or a C₁-C₆ linear alkyl group, R³ represents a C₂-C₄₀ linear, branched, or cyclic alkyl group, R⁴CO- represents a group derived from a carboxylic acid halide or an acid anhydride, and n represents a number between 1 and 16 inclusive that includes:
reacting an aminocarboxylic acid ester salt having the formula (3): 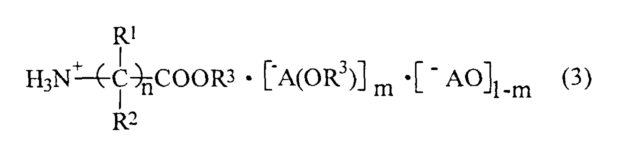
wherein R¹ and R², which may be identical to or different from each other, represent a hydrogen atom or a C₁-C₆ linear alkyl group, R³ represents a C₂-C₄₀ linear, branched, or cyclic alkyl group, ⁻A(OR³), wherein R³ has the same meaning as described above, represents an inorganic acid ester from which a single hydrogen ion has been released, ⁻AO represents a divalent or trivalent acid residue from which a single hydrogen ion has been released, n represents a number between 1 and 16 inclusive, and m represents a number between 0 and 1 inclusive, with a carboxylic acid halide or an acid anhydride of formula R⁴COX wherein X is a halogen atom or an acid anhydride residue (R⁴COO-) in the presence of a tertiary amine.

### DESCRIPTION OF THE PREFERRED EMBODIMENTS

A more complete appreciation of the invention and many of the attendant advantages thereof will be readily obtained as the same becomes better understood by reference to the following detailed description of the preferred embodiments, which are not intended to be limiting.
The process of the present invention is preferably represented by the following reaction scheme. 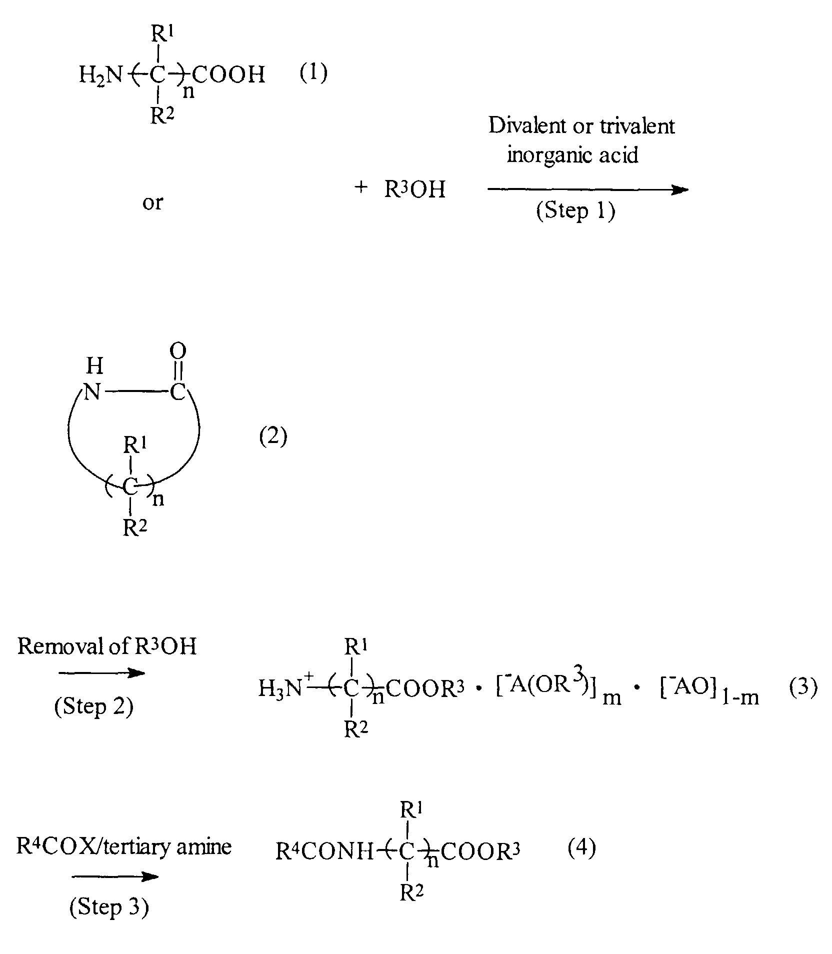 wherein each of R¹ and R², which may be identical to or different from each other, represents a hydrogen atom or a C₁-C₆ linear alkyl group, R³ represents a C₂-C₄₀ linear, branched, or cyclic alkyl group, R⁴CO represents a group derived from a carboxylic acid halide or an acid anhydride, X represents a halogen atom or an acid anhydride residue (R⁴COO-), ⁻A(OR³) (wherein R³ has the same meaning as described above) represents an inorganic acid ester from which a single hydrogen ion has been released, ⁻AO represents a divalent or trivalent acid residue from which a single hydrogen ion has been released, n represents a number between 1 and 16 inclusive, and m represents a number between 0 and 1 inclusive.

Accordingly, the present invention provides a process for the manufacture of an amide derivative of formula (4), that includes the steps of:
reacting in the presence of a divalent or trivalent inorganic acid an aminocarboxylic acid of formula (1) or a cyclic lactam of formula (2) with a monohydric alcohol having 2 or more carbon atoms,
removing unreacted alcohol, and subsequently reacting the resultant product with a carboxylic acid halide or an acid anhydride in the presence of a tertiary amine.

The present invention also provides a derivative of an aminocarboxylic ester salt of formula (3) and a process for manufacture of the derivative.

In formula (1), the C₁-C₆ linear alkyl groups represented by R¹ and R² are not particularly limited, and any species that corresponds to the amide derivative (4) of interest may be used. In consideration of reactivity and yield, a hydrogen atom, a methyl group, and an ethyl group are preferred. R¹ and R², respectively being present in the number of n, may be identical to or different from each other.

The number n, though not particularly limited, is preferably between 2 and 12 inclusive from the viewpoint of reactivity and yield of the amide derivative (4).

The C₂-C₄₀ linear, branched, or cyclic alkyl groups represented by R³ are not particularly limited, and they are preferably C₂-C₁₂ linear, branched, or cyclic alkyl groups, more preferably C₂-C₈ linear, branched, or cyclic alkyl groups, and most preferably C₂-C₈ branched alkyl groups. Examples of such C₂-C₄₀ linear, branched, or cyclic alkyl groups represented by R³ are ethyl, propyl and cyclopropyl groups, and linear, branched, or cyclic groups selected from butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, octacosyl, nonacosyl, triacontyl, hentriacontyl, dotriacontyl, tritriacontyl, tetratriacontyl, pentatriacontyl, hexatriacontyl, heptatriacontyl, octatriacontyl, nonatriacontyl and tetracontyl groups.

Examples of the acid residue represented by ⁻AO include a sulfate residue (⁻HSO₄) and a phosphate residue (⁻H₂PO₄), with the sulfate residue being preferred.

The group represented by ⁻A(OR₃) denotes an inorganic acid ester from which a single hydrogen ion has been released. Specifically, this group represents a residual group corresponding to an inorganic acid residue of an ester formed from a reaction of a divalent or trivalent inorganic acid (AOH) and R³OH (R³ has the same meaning as described above), from which inorganic acid residue a single hydrogen ion has been eliminated. Examples of the divalent or trivalent inorganic acids (AOH) include those which produce the aforementioned ⁻AO as a result of removal of hydrogen. Illustrative examples thereof include sulfuric acid (H₂SO₄) and phosphoric acid (H₃PO₄), with sulfuric acid being preferred. Examples of R³ have already been described hereinabove.

m is a number between 0 and 1 inclusive, and is generally considered to be between 0.5 and 1 inclusive.

R⁴ is preferably a C₁-C₄₀ hydrocarbon group, and more preferably a C₁-C₂₂ linear, branched, or cyclic hydrocarbon group. Examples of such C₁-C₄₀ hydrocarbon groups for R⁴ are are methyl, ethyl, propyl and cyclopropyl groups, and linear, branched, or cyclic groups selected from butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, octacosyl, nonacosyl, triacontyl, hentriacontyl, dotriacontyl, tritriacontyl, tetratriacontyl, pentatriacontyl, hexatriacontyl, heptatriacontyl, octatriacontyl, nonatriacontyl and tetracontyl groups. The C₁-C₄₀ hydrocarbon group for R⁴ may be substituted or unsubstituted. X represents a halogen atom such as chlorine, bromine, and iodine, or an acid anhydride residue (R⁴COO-).

Each preferred step of the process of the present invention will be described.

### (Step 1)

In Step 1, an aminocarboxylic acid (1) or a cyclic lactam (2) is reacted with a monohydric alcohol of R³OH in the presence of a divalent or trivalent inorganic acid.

Preferred examples of the aminocarboxylic acid (1) used in the process of the present invention include 6-aminohexanoic acid, 11-aminoundecanoic acid, and 12-aminododecanoic acid.

Preferred examples of the cyclic lactam (2) include ε-caprolactam, undecalactam, and laurinlactam.

The alcohol (R³-OH) which is used in the present invention is preferably C₂-C₁₂ linear, branched, or cyclic alcohol, more preferably C₂-C₈ linear, branched, or cyclic alcohol, most preferably C₂-C₈ branched alcohol, from the viewpoint that the resultant aminocarboxylic acid ester salt derivative (3) is liquid or is freely soluble in a solvent, and also in consideration of reactivity, yield, and ease in removal after completion of reaction. Examples of such C₂-C₁₂ linear, branched, or cyclic alcohols for R³-OH are ethanol, propanol and cyclopropanol, and linear, branched or cylic alcohols selected from butanol, pentanol, hexanol, heptanol, octanol, nonanol, decanol, undecanol and dodecanol.

Examples of the divalent or trivalent inorganic acids include sulfuric acid and phosphoric acid. From the viewpoint that the resultant aminocarboxylic ester salt can be maintained in a dissolved or liquid state, sulfuric acid is preferred.

The reaction is performed in the absence of a solvent or in an inert solvent.

Examples of the inert solvent include hydrocarbon solvents such as hexane; aromatic solvents such as benzene, toluene, and xylene; and ether solvents such as diethylether and tetrahydrofuran.

This step is carried out under ambient pressure or reduced pressure. If necessary, the reaction mixture may be heated. The reaction temperature is from room temperature to 200°C, preferably from 50°C to the boiling point of the monohydric alcohol at the reaction pressure.

### (Step 2)

In Step 2, the excess alcohol contained in the reaction mixture obtained in Step 1 is preferably removed by distillation. This process is carried out under ambient pressure or reduced pressure, and if necessary, heat may be applied or the reaction system is cooled. The reaction temperature is from 0°C to 200°C, preferably from 20°C to the boiling point of the monohydric alcohol at the reaction pressure.

Through this step, the aminocarboxylic acid ester salt derivative (3) of the present invention is obtained.

### (Step 3)

In Step 3, the aminocarboxylic acid ester salt derivative (3) of the present invention obtained in Step 2 is reacted with carboxylic acid halide or an acid anhydride (R⁴COX) in an inert solvent in the presence of a tertiary amine.

Examples of the carboxylic acid halides include acetic chloride, propionic chloride, butanoic chloride, hexanoic chloride, octanoic chloride, decanoic chloride, dodecanoic chloride, tetradecanoic chloride, hexadecanoic chloride, behenic chloride, isobutanoic chloride, 2-ethylhexanoic chloride, isononanoic chloride, and isostearic chloride. Examples of the acid anhydride include acetic anhydride.

Examples of the tertiary amine include trimethylamine, triethylamine, tripropylamine, tributylamine, pyridine, and dimethylpyridine.

Examples of the inert solvent include hexane, toluene, xylene, benzene, diethylether, tetrahydrofuran, methylene chloride, and chloroform.

This step is carried out within a temperature range from 0°C to 100°C, preferably from 10°C to 80°C.

After completion of reaction, the tertiary amine salt produced in the reaction mixture is removed by washing or by similar means, and subsequently, the solvent is distilled off to thereby yield N-acylaminocarboxylic acid ester with ease.

Since the aminocarboxylic acid ester salt (3) produced on the way of the manufacture of amide derivatives is in a liquid state or is freely soluble in a solvent, the process of manufacture of amide derivatives according to the present invention does not form a solid compound, and thus the amide derivatives can be produced industrially with ease. Moreover, the entire acylation process is industrially advantageous.

### Examples

Having generally described this invention, a further understanding can be obtained by reference to certain specific examples which are provided herein for purposes of illustration only and are not intended to be limiting unless otherwise specified.

### Example 1:

(1) 12-Aminododecanoic acid (100 g) was charged in a 1-liter flask. Isobutanol (344 g) was added and stirring was initiated. Subsequently, the flask was heated to 50°C, and sulfuric acid (52.2 g) was added dropwise. After completion of addition, the contents of the flask were refluxed with heat, and five hours later, they were cooled to 70°C. When isobutanol was distilled off under reduced pressure, a liquid compound, 12-aminododecanoic acid isobutyl ester sulfate isobutyl sulfate, was obtained.
   NMR: (ppm, in CDCl₃, reference: CHCl₃)
      0.70 (d, J=6.6Hz, 6H), 0.80 (d, J=6.6Hz, 6H), 1.12-1.49 (m, 14H), 1.49-1.82 (m, 4H), 1.82-2.12 (m, 2H), 2.32 (t, J=7.5Hz, 2H), 2.82-3.18 (m, 2H), 3.78 (d, J=6.6Hz, 2H), 3.87 (d, J=6.6Hz, 2H)
   IR (cm⁻¹, KBr)
      2932, 1740, 1473, 1236, 1203, 1056, 996, 882, 846, 804, 582
(2) Subsequently, toluene (300 g) was added to the resultant liquid and dissolved. The solution was cooled to 40°C, and triethylamine (155 g) was added dropwise for 30 minutes. Acetic anhydride (52 g) was added dropwise over 1 hour, and the reaction was allowed to proceed for 5 hours. After completion of reaction, the salt that had been produced was washed with water. The solvent was distilled off under reduced pressure, to thereby obtain 12-acetylaminododecanoic acid isobutyl ester (131 g).

### Example 2:

12-Aminododecanoic acid (100 g) was charged in a 1-liter flask. Ethanol (344 g) was added and stirring was initiated. Subsequently, the flask was heated to 50°C, and sulfuric acid (52.2 g) was added dropwise. After completion of addition, the contents of the flask were refluxed with heat, and five hours later, they were cooled to 70°C. When ethanol was distilled off under reduced pressure, a liquid compound, 12-aminododecanoic acid ethyl ester sulfate ethyl sulfate, was obtained. Subsequently, toluene (300 g) was added to the liquid and dissolved. The solution was cooled to 40°C, and triethylamine (155 g) was added dropwise over 30 minutes. Dodecanoic chloride (52 g) was added dropwise over 1 hour, and the reaction was allowed to proceed for 5 hours. After completion of reaction, the salt that had been produced was washed with water. The solvent was distilled off under reduced pressure, to thereby obtain 12-dodecanoylaminododecanoic acid ethyl ester (180 g).

### Example 3:

12-Aminododecanoic acid (100 g) was charged in a 1-liter flask. 2-Ethylhexanol (344 g) was added and stirring was initiated. Subsequently, the flask was heated to 50°C, and sulfuric acid (52.2 g) was added dropwise. After completion of addition the contents of the flask were refluxed with heat, and five hours later, they were cooled to 70°C. When 2-ethylhexanol was distilled off under reduced pressure, a liquid compound, 12-aminododecanoic acid 2-ethylhexyl ester sulfate 2-ethylhexyl sulfate, was obtained. Subsequently, toluene (300 g) was added to the liquid and dissolved. The solution was cooled to 40°C, and triethylamine (155 g) was added dropwise over 30 minutes. Hexanoic chloride (52 g) was added dropwise over 1 hour, and the reaction was allowed to proceed for 5 hours. After completion of reaction, the salt that had been produced was washed with water. The solvent was distilled off under reduced pressure, to thereby obtain 12-hexanoylaminododecanoic acid 2-ethylhexyl ester (164 g).

### Example 4:

ε-Caprolactam (100 g) was charged in a 1-liter flask. Isobutanol (344 g) was added and stirring was initiated. Subsequently, the flask was heated to 50°C, and sulfuric acid (52.2 g) was added dropwise. After completion of addition, the contents of the flask were refluxed with heat, and five hours later, they were cooled to 70°C. When isobutanol was distilled off under reduced pressure, a liquid compound, 6-aminocaproic acid isobutyl ester sulfate isobutyl sulfate, was obtained. Subsequently, toluene (300 g) was added to the liquid and dissolved. The solution was cooled to 40°C, and triethylamine (155 g) was added dropwise over 30 minutes. Methyl-branched isostearic chloride (52 g) was added dropwise over 1 hour, and the reaction was allowed to proceed for 5 hours. After completion of reaction, the salt that had been produced was washed with water. The solvent was distilled off under reduced pressure, to thereby obtain methyl-branched isostearoylaminocaproic acid isobutyl ester (196 g).

Obviously, numerous modifications and variations of the present invention are possible in light of the above teachings. It is therefore to be understood that within the scope of the appended claims, the invention may be practiced otherwise than as specifically described herein.

The application is based on Japanese Patent Applications 329728/1996 and 329729/1996, each filed December 10, 1996, the entire contents of which are hereby incorporated by reference.

## Claims

1. A process for preparing an N-acylaminocarboxylic acid ester of the formula (4): 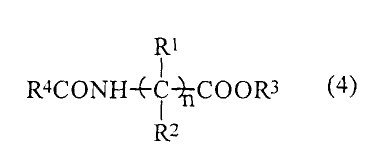 wherein R¹ and R², which may be identical to or different from each other, represent a hydrogen atom or a C₁-C₆ linear alkyl group, R³ represents a C₂-C₄₀ linear, branched, or cyclic alkyl group, R⁴CO- represents a group derived from a carboxylic acid halide or an acid anhydride, and n represents a number between 1 and 16 inclusive, comprising:
reacting an aminocarboxylic acid of formula (1): 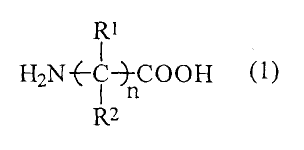
wherein R¹, R², and n have the same meanings as defined above,
or a cyclic lactam of formula (2): 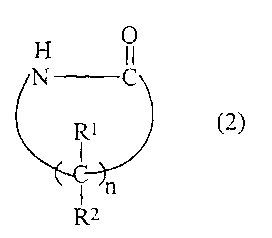
wherein R¹, R², and n have the same meanings as defined above with a monohydric alcohol (R³OH) having 2-40 carbon atoms in the presence of a divalent or trivalent inorganic acid,
removing unreacted alcohol thereby forming a resultant product; and
reacting the resultant product with a carboxylic acid halide or an acid anhydride of formula R⁴COX wherein X is a halogen atom or an acid anhydride residue (R⁴COO-) in the presence of a tertiary amine.

2. The process according to Claim 1, wherein the divalent or trivalent inorganic acid is sulfuric acid or phosphoric acid.

3. The process according to Claim 1, wherein R³ is a C₂-C₈ linear, branched, or cyclic alkyl group.

4. The process according to Claim 1, wherein the aminocarboxylic acid of formula (1) is 12-aminododecanoic acid, 11-aminoundecanoic acid, or 6-aminohexanoic acid.

5. The process according to Claim 1, wherein the cyclic lactam of formula (2) is ε-caprolactam, undecalactam, or laurinlactam.

6. The process according to Claim 1, wherein R⁴ in the carboxylic acid halide or the acid anhydride (R⁴COX) is a hydrocarbon group having 1-40 carbon atoms.

7. The process according to Claim 1, wherein said resultant product is an aminocarboxylic acid ester salt having the formula (3): 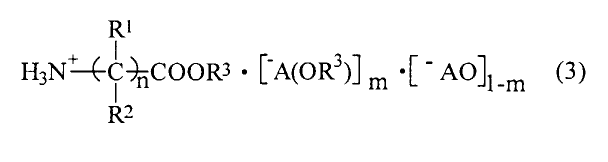 wherein R¹ and R², which may be identical to or different from each other, represent a hydrogen atom or a C₁-C₆ linear alkyl group, R³ represents a C₂-C₄₀ linear, branched, or cyclic alkyl group, ⁻A(OR³), wherein R³ has the same meaning as described above, represents an inorganic acid ester from which a single hydrogen ion has been released, ⁻AO represents a divalent or trivalent acid residue from which a single hydrogen ion has been released, n represents a number between 1 and 16 inclusive, and m represents a number between 0 and 1 inclusive.

8. An aminocarboxylic acid ester salt having the formula (3): 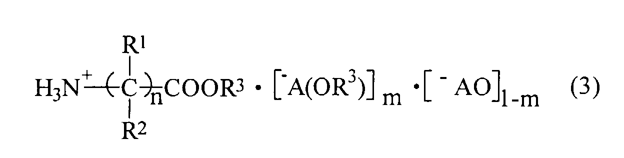 wherein R¹ and R², which may be identical to or different from each other, represent a hydrogen atom or a C₁-C₆ linear alkyl group, R³ represents a C₂-C₄₀ linear, branched, or cyclic alkyl group, ⁻A(OR³), wherein R³ has the same meaning as described above, represents an inorganic acid ester from which a single hydrogen ion has been released, ⁻AO represents a divalent or trivalent acid residue from which a single hydrogen ion has been released, n represents a number between 1 and 16 inclusive, and m represents a number between 0 and 1 inclusive.

9. The aminocarboxylic acid ester salt according to Claim 8, wherein ⁻AO is a sulfate residue ⁻HSO₄ or a phosphate residue ⁻H₂PO₄

10. The aminocarboxylic acid ester salt according to Claim 8, wherein R³ is a C₂-C₈ linear, branched, or cyclic alkyl group.

11. A process for preparing an aminocarboxylic acid ester salt having the formula (3): 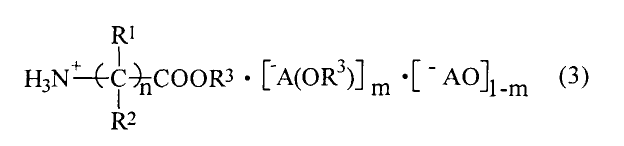 wherein R¹ and R², which may be identical to or different from each other, represent a hydrogen atom or a C₁-C₆ linear alkyl group, R³ represents a C₂-C₄₀ linear, branched, or cyclic alkyl group, ⁻A(OR³), wherein R³ has the same meaning as described above, represents an inorganic acid ester from which a single hydrogen ion has been released, ⁻AO represents a divalent or trivalent acid residue from which a single hydrogen ion has been released, n represents a number between 1 and 16 inclusive, and m represent a number between 0 and 1 inclusive, comprising:
reacting an aminocarboxylic acid of formula (1): 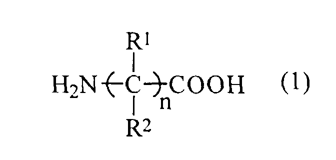
wherein R¹ and R², which may be identical to or different from each other, represent a hydrogen atom or a C₁-C₆ linear alkyl group, and n represents a number between 1 and 16 inclusive,
or a cyclic lactam of formula (2): 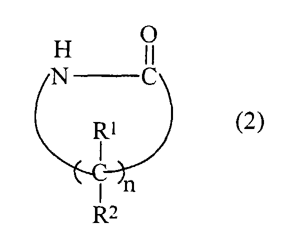
wherein R¹, R², and n have the same meanings as defined above, with a monohydric alcohol R³OH having 2-40 carbon atoms in the presence of a divalent or trivalent inorganic acid; and
removing unreacted alcohol.

12. The process according to Claim 11, wherein the aminocarboxylic acid of formula (1) is 12-aminododecanoic acid, 11-aminoundecanoic acid, or 6-aminohexanoic acid.

13. The process according to Claim 11, wherein the cyclic lactam of formula (2) is ε-caprolactam, undecalactam, or laurinlactam.

14. A process for preparing an N-acylaminocarboxylic acid ester of the formula (4): 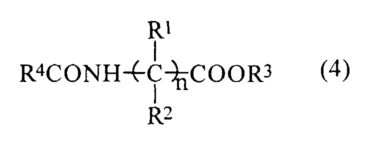 wherein R¹ and R², which may be identical to or different from each other, represent a hydrogen atom or a C₁-C₆ linear alkyl group, R³ represents a C₂-C₄₀ linear, branched, or cyclic alkyl group, R⁴CO- represents a group derived from a carboxylic acid halide or an acid anhydride, and n represents a number between 1 and 16 inclusive, comprising:
reacting an aminocarboxylic acid ester salt having the formula (3): 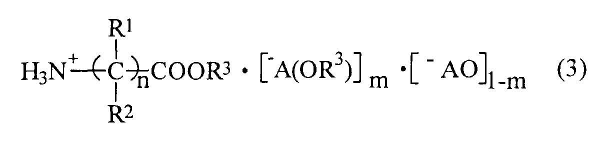
wherein R¹ and R², which may be identical to or different from each other, represent a hydrogen atom or a C₁-C₆ linear alkyl group, R³ represents a C₂-C₄₀ linear, branched, or cyclic alkyl group, ⁻A(OR³), wherein R³ has the same meaning as described above, represents an inorganic acid ester from which a single hydrogen ion has been released, ⁻AO represents a divalent or trivalent acid residue from which a single hydrogen ion has been released, n represents a number between 1 and 16 inclusive, and m represents a number between 0 and 1 inclusive, with a carboxylic acid halide or an acid anhydride of formula R⁴COX wherein X is a halogen atom or an acid anhydride residue (R⁴COO-) in the presence of a tertiary amine.

15. The process according to Claim 14, wherein R³ is a C₂-C₈ linear, branched, or cyclic alkyl group.

16. The process according to Claim 14, wherein R⁴ in the carboxylic acid halide or the acid anhydride (R⁴COX) is a hydrocarbon group having 1-40 carbon atoms.
